# NEUE EUROPÄISCHE PATENTSCHRIFT

(11) **EP 0 576 509 B2**
(45) Veröffentlichungstag und Bekanntmachung des Hinweises auf die Entscheidung über den Einspruch: **14.11.2007**
(45) Hinweis auf die Patenterteilung: 03.07.1996
(21) Anmeldenummer: 92906803.9
(22) Anmeldetag: 18.03.1992
(51) Int. Cl.: A61N 1/365

(54) **MEDIZINISCHES GERÄT ZUR ERZEUGUNG EINER BEHANDLUNGSGRÖSSE**
MEDICAL APPLIANCE FOR GENERATING A THERAPEUTICAL PARAMETER
APPAREIL MEDICAL DE GENERATION D'UN PARAMETRE THERAPEUTIQUE

(30) Priorität: 18.03.1991 DE 4109202
(43) Veröffentlichungstag der Anmeldung: 05.01.1994
(73) Patentinhaber: Biotronik GmbH & Co. KG, 12359 Berlin (DE)
(72) Erfinder: SCHALDACH, Max, D-8520 Erlangen (DE); HASTINGS, David, Micro Systems Engineering, Inc., Lake Oswego, OR 97035 (US); KULP, Barry D., Micro Systems Engineering, Inc., Lake Oswego, OR 97035 (US)
(74) Vertreter: Eisenführ, Speiser & Partner
(86) Internationale Anmeldenummer: PCT/DE1992/000238
(87) Internationale Veröffentlichungsnummer: WO 1992/016256

(56) Entgegenhaltungen:
- EP-A- 0 215 731
- EP-A- 0 225 839
- EP-A- 0 228 985
- EP-A- 0 232 528
- EP-A- 0 259 658
- EP-A- 0 311 019
- GB-A- 2 070 282
- US-A- 4 280 502
- US-A- 4 992 907
- Max Schaldach: "Automatic Adjustment of Pacing Parameters Based on Intracardiac Impedance Measurements" from Pacing and Clinical Electrophysiology (PACE) Vol. 13, December 1990, Part II, pages 1702-1710

## Beschreibung

Die Erfindung betrifft ein medizinisches Gerät der im Oberbegriff des Anspruchs 1 angegebenen Art.

Bei derartigen Geräten, weiche eine Behandlungsgröße für einen Patienten aus einer im Körper des Patienten aufgenommenen Größe (Parameter) erzeugen, besteht vielfach das Problem, daß dieser Parameter, mit dessen Hilfe die Behandlung des Patienten vorgenommen wird, von einem weiteren Parameter abhängt, über den im Zusammenhang mit der vorgesehenen Behandlung keine soweit gesicherten Erkenntnisse vorliegen, daß er im Rahmen der Behandlung als weitere Variable eingesetzt werden kann oder der aus anderen Gründen außer Betracht bleiben muß. Da der weitere Parameter somit jeweils einen relativ zufälligen Wert einnimmt, kann ein Behandlungserfolg nicht mit der notwendigen Zuverlässigkeit sichergestellt werden.

Gattungsgemäße Geräte sind beispielsweise in EP-A-0 222 681 und EP-A-0 228 985 beschrieben.

EP-A-0 222 681 beschreibt einen Herzschrittmacher, bei dem Mittel zur Erfassung und Zusammenführung mehrerer, von der körperlichen Belastung abhängiger, Meßgrößen im Körper eines Patienten zur Gewinnung einer. Behandlungsgröße (der Stimulationsrate) vorgesehen sind. Im Hinblick darauf, wie bei Vorliegen einer Abhängigkeit eines im Körper aufgenommenen Parameters von einem weiteren Parameter eine hinsichtlich der Empfindlichkeit und Reproduzierbarkeit optimierte Nutzung des ersteren als Steuergröße ermöglicht werden kann, trifft das Dokument keinen Aussage.

EP-A-0 228 985 beschreibt einen Schrittmacher mit Mitteln zur Zuordnung einer Behandlungsgröße zu einem im Körper aufgenommenen Parameter und zu einer multiplikativen Verknüpfung dieser beiden Größen zur Gewinnung einer zusätzlichen - synthetischen - Bezugsgröße für die Steuerung der Behandlungsgröße. Das Dokument enthält keine Hinweise auf die Handhabung eines zusätzlichen Parameters, der den im Körper aufgenommenen Parameter beeinflußt.

Aus GB-A-2 070 282 ist ein Herzschrittmacher bekannt, der die Impedanz im Herzen mittels zweier Elektroden mißt und daraus ein Signal als Steuergröße für die Herzrate ableitet. Dazu wird das Analogsignal der elektrischen Impedanz bezüglich seiner Spitzen ausgewertet Der ermittelte Spitzen-Spitzen-Wert wird mit dem zuvor ermittelten Spitzen-Spitzen-Wert verglichen und die Herzrate dem Vergleichsergebnis entsprechend verändert.

Aus US 4,733,667 ist ein Herzschrittmacher bekannt, bei dem ein Steuersignal für die Ratensteuerung aus der Veränderungsrate dZ/dt des Impedanzverlaufs mittels eines Differentiators abgeleitet wird.

Gemäß Max Schaldach "Automatic Adjustment of Pacing Parameters Based on Intracardiac Impedance Measurements", PACE, Band 13, Seiten 1702-1710, Dezember 1990, wird der intrakardiale Impedanzverlauf zur Bestimmung des Endes der Pre Ejection Period (PEP) ausgewertet und eine Ratensteuerung basierend auf der Dauer der PEP als Ratensteuerparameter vorgeschlagen.

So konnte bei Herzschrittmachern bisher noch keine sichere Regel angegeben werden, um die Stimulationsrate in Abhängigkeit von zu bestimmten Zeitpunkten innerhalb des Herzzyklus im Rahmen der Pre-ejectionperiod im Herzen aufgenommenen Leitfähigkeitswerten mit maximaler Effektivität zu beeinflussen.

Die erhaltenen Werte streuten stark von Patient zu Patient, so die Übertragung von bei einem Patienten gefundener Einstellungen auf einen anderen Patienten zunehmend mit Schwierigkeiten verbunden war.

Ausgewertet wurde dabei meist die Steigung des Leitwerts, ermittelt durch im rechten Ventrikel verlegte Elektroden, welche bevorzugterweise gleichzeitig Elektroden des Stimulationssystems bilden können.

Der Erfindung liegt die Aufgabe zugrunde, bei einem medizinischen Gerät der eingangs genannten Gattung die Möglichkeit zu schaffen, die Erfassungsbedingungen für den eine Behandlungsgröße steuernden, im Körper des Patienten aufzunehmenden Parameter so zu beeinflussen, daß die Empfindlichkeit der Steuerung oder Regelung ein Maximum wird und sich insbesondere die bei verschiedenen Patienten anzuwendenden Regelungsalgorithmen auf andere Patienten übertragen lassen.

Diese Aufgabewird mit den kennzeichnenden Merkmalen des Anspruchs 1 gelöst.

Der Erfindung liegt die Erkenntnis zugrunde, daß vielfach ein oder mehrere weitere(r) Parameter vorhanden ist sind, welche(r) die Erfassung des auszuwertenden Parameters unerkannt beeinflußt/beeinflussen. Wenn es nun gelingt, diese(n) Parameter bei der Messung so einzustellen, daß die Änderung des auszuwertenden Parameters im vorgesehenen Behandlungsbereich des Patienten ein Maximum wird, so kann in vielen Fällen die zur Veränderung der für die Behandlung des Patienten zu beeinflussenden Größe zur Verfügung stehende Information wesentlich verbessert bzw. erst erschlossen werden.

Es Wird also jeweils ein weiterer Parameter mittels geeigneter Maßnahmen so verändert, daß die Beeinflussung körperlicher Vorgänge des Patienten in Abhängigkeit von einer ersten vom Körper abgeleiteten Größe maximiert ist, so daß ein maximales Ansprechen auf die Behandlung gewährleistet ist. Diese Maximierung der Beeinflussbarkeit des Patienten wird in einem "Lernzyklus" des Geräts festgestellt und das ermittelte Ergebnis dann für die weitere Behandlung angewendet.

Bei dem gattungsgemäßen medizinischen Gerät zur Erzeugung einer Behandlungsgröße für einen Patienten in Abhängigkeit von mindestens einem innerhalb des Körpers aufgenommenen ersten Parameter wird also zunächst unterstellt, daß der erste Parameter von einem weiteren (zweiten) Parameter abhängig ist. Hierbei kann es sich beispielsweise um ein (auch zeitweise) Maskierung des für die Veränderung der Behandlungsgröße wesentlichen, im Körper des Patienten aufzunehmenden Signals (erster Parameter) handeln.

Wenn sich der erste Parameter mit äußeren Maßnahmen gezielt verändern läßt, so wird der bei der Behandlung des Patienten wesentliche Veränderungsbereich des ersten Parameters durchlaufen. Wenn sich also beispielsweise alle Bedingungen des täglichen Lebens des Patienten, unter denen eine Behandlung - insbesondere mit tragbaren oder implantierbaren medizinischen Geräten · erfolgen soll, simulieren oder einstellen lassen, so kann die entsprechende Veränderung des ersten Parameters verfolgt werden.

Mit den Maßnahmen der Erfindung erfolgt nun die Erzeugung der BehandlungsgröBe unter Variation des zweiten kontrollierbaren Parameters derart, daß die Differenz der Werte des ersten Parameters an den Grenzen des Variationsbereichs des ersten Parameters ein Maximum bildet.

Der verändernde weitere Parameter bei der Signalautnahme im Körper des Patienten stellt das Zeitfenster dar, zu dem die Signalaufnahme im Rahmen eines zyklischen Ablaufs, dem Herzrhythmus erfolgt.

Diese Einstellung des zweiten kontrollierbaren Parameters wird nun in einem Speicher festgehalten und bei der weiteren Beeinflussung der Behandlungsgröße des Patienten in Abhängigkeit von dem ersten Parameter zugrundegelegt, so daß die Steuerung der Behandlungsgröße innerhalb des Variationsbereichs des ersten Parameters unter Beibehaltung des so gewonnenen zweiten Parameters erfolgt.

Bei einer anderen vorteilhaften Weiterbildung der

Erfindung werden für verschiedene Variationsbereiche des ersten Parameters unterschiedliche, jeweils einem derartigen Variationsbereich zuzuordnende Werte des zweiten Parameters ermittelt und in dem Speicher festgehalten. Jetzt kann - auch wenn der Einfluß des zweiten Parameters auf die Abhängigkeit der den Patienten beeinflussenden Größe vom ersten Parameter über den Veränderungsbereich des ersten Parameters nicht unverändert bleibt, durch entsprechende Umschaltung der Auswahl des zweiten Parameters jeweils abschnittsweise die Steuerung bzw. Regelung der den Patienten beeinflussenden Größe optimiert werden. Die Umschaltung der Beeinflussung des zweiten Parameters kann dabei über zusätzliche Mittel erfolgen, von einem dritten Parameter verändert werden, welcher sich in seiner Tendenz wie der erste Parameter verändert und ebenfalls im Körper des Patienten oder in seiner Umgebung ableitbar ist.

Bei einer anderen bevorzugten Ausführungsform kann die Umschaltung der Auswahl des zweiten Parmeters auch aufgrund des ersten Parameters selbst erfolgen, wenn dafür Sorge getragen ist, daß durch eine Schalthysterese oder andere geeignete Maßnahmen für die notwendige Stabilität der Steuerung gesorgt ist und Regelschwingungen nicht auftreten können.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt Es zeigen:
Figur 1 einen Herzzyklus-Signal mit einem Zeitfenster, in dem die Signal-Aufnahme erfolgt,
Figur 2 ein Ausführungsbeispiel einer Schaltung zur bedarfsabhängigen Veränderung der Stimulationsrate bei einem künstlichen Herzschrittmacher sowie

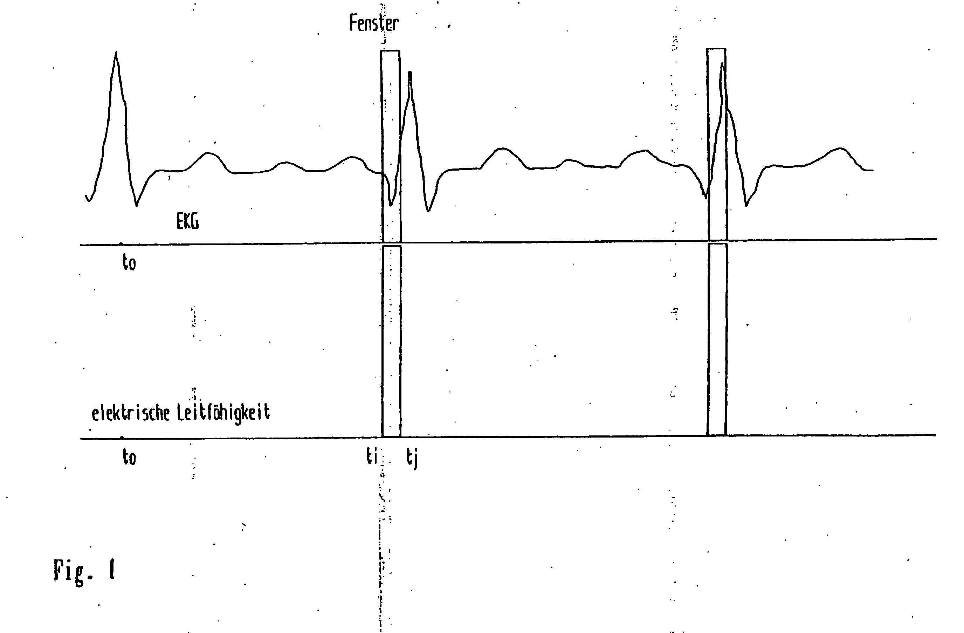

Die Erfindung wird in vorteilhafter Weise angewendet auf implantierbare Herzschrittmacher, welche mit einer Schaltung zur bedarfsabhängigen Veränderung der Stimulationsrate versehen sind. Die mittels der Stimulationselektrode E₁ gemessene elektrische Leitfähigkeit κ im Herzen wird in Figur 1 als Herzzyklus mit Zeitfenster gezeigt.

Es bildet insbesondere die Veränderung der elektrischen Leitfähigkeit im Herzen, als erster Parameter, ein Maß für die körperliche Belastung, welche eine entsprechende Pumpleistung (Minutenvolumen) des Herzens erfordert. Diese Größe ist unabhängig davon, ob es sich um einen geschlossenen Regelkreis oder eine Steuerung handelt, eine Eingangsgröße für die Schaltung zur bedarfsabhängigen Veränderung der Stimulationsrate 10. Die Stimulationsrate des Schrittmachers (bei Bedarfsschrittmachern die Grundrate) wird von einer Steuer- und/oder Regeleinrichtung 3 in Abhängigkeit von der für die körperliche Belastung repräsentativen Größe verändert.

Diese repräsentative Größe ist die Steigung S der elektrischen Leitfähigkeit κ vorzugsweise im rechten Ventrikel, d.h. die Differenz der elektrischen Leitfähigkeit Δκ am Anfang und Ende eines bestimmten vorgebbaren Zeitintervalls Δtᵢⱼ.

Dafür wird ein Zeitfenster Zᵢⱼ = f(Δtᵢ, Δtⱼ) von einem Speicher 6 vorgegeben und bildet den zweiten Parameter, der das Meßsignal beeinflußt Von der Eingangsgröße wird in einer Eingangsschaltung 1 ein fester zeitlicher Bezugspunkt tₒ abgeleitet. Somit ist Δtᵢⱼ das vorwählbare Zeitintervall, welches zum Zeitpunkt tᵢ, d.h. im Abstand Δtᵢ vom Bezugspunkt tₒ, beginnt und im Abstand Δtⱼ vom Bezugspunkt tₒ endet. Es wurden bisher viele vergebliche Versuche unternommen, ein jeweils geeignetes Zeitfenster zu finden, welches mit genügender Genauigkeit reproduzierbare Ergebnisse und insbesondere auch eine Übertragung auf andere Patienten ermöglicht.

Die Veränderung (Steigung) des Kurzzeitintegrals der elektrischen Leitfähigkeit innerhalb eines bestimmten Zeitfensters im Herzzyklus bildet somit mindestens mittelbar eine Eingangsgröße für die Schaltung zur bedarfsabhängigen Veränderung der Stimulationsrate 10.

Gemäß einer vorteilhaften Weiterbildung der Erfindung wird nun dasjenige Zeitintervall (Zeitfenster) ermittelt und zur späteren Differenzbildung (Steigungsermittlung) der gemessenen Leitfähigkeitswerte herangezogen, bei dem der Unterschied für zwei verschiedene Belastungszustände des Patienten ein Maximum bildet.

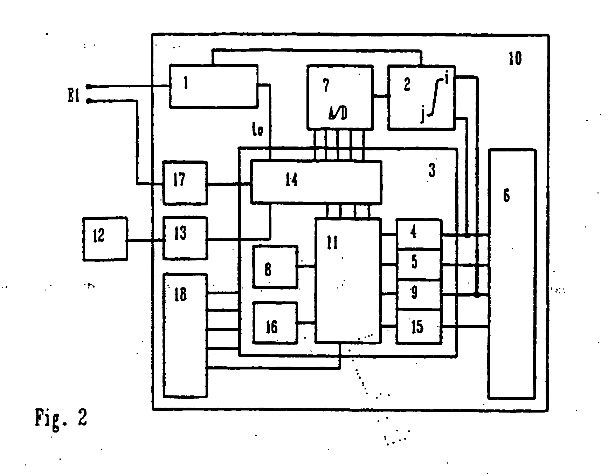

Für das Ausführungsbeispiel des implantierbaren Herzschrittmachers ist in Figur 2 eine Schaltung zur bedarfsabhängigen Veränderung der Stimulationsrate 10 dargestellt. Die im Herzen mittels der Stimulationselektrode E₁ gemessene elektrische Leitfähigkeit κ wird in einer ersten Eingangsschaltung 1 und zweiten Eingangsschaltung 2 verarbeitet und bildet eine Eingangsgröße für eine Steuer- und/oder Regeleinrichtung 3 in der Schaltung zur bedarfsabhängigen Veränderung der Stimulationsrate 10.

Das über geeignete Aufnahmepunkte an der Stimulationselektrode E1 im rechten Ventrikel ermittelte und von der ersten zur zweiten Eingangsschaltung 2 übermittelte verstärkte Leitfähigkeitssignal wird in der zweiten Eingangsschaltung 2 in dem Zeitintervall integriert, um kurzzeitige, das Meßsignal verfälschende Schwankungen auszuschalten.

Die Eingangsschaltung 2 ist über einen Anatog/Digital-Umsetzer 7 und die Eingangsschaltung 1 mit der Steuer- und/oder Regeleinrichtung 3, insbesondere mit ihrem Ein-/Ausgabe-Kanal 15, verbunden. Diese enthält neben dem Ein-/Ausgabe-Kanal 15 eine Logik bzw. vorzugsweise einen Prozessor 11, außerdem für den Speicheranschluß geeignete Auswahlschaltungen 4, 5, 9 und eine Datenein-/ausgabe-Schaltung 15 für die Steigungsgrenzwerte, eine die Steuerung des Variationsbereiches vornehmende Ratenauswahlschaltung 8 und einen Taktgeber 16.

Die Steuer- und/oder Regelschaltung 3 ist einerseits mit ihren zwei Schaltungen 4 und 9 zur Auswahl der Zeitintervalle mit der zweiten Eingangsschaltung 2 sowie mit einer Speicherbereichsauswahlschaltung 5 und mit einem Speicher 6 verbunden. In weiterer Ausgestaltung ist am Speicher 6 zur Speicherung von Grenzwerten für den ersten Parameter auch die Daten-Ein/Ausgabe-Schaltung 15 angeschlossen. Andererseits ist die Steuer- und/oder Regelschaltung 3 mit ihrem Ein-/Ausgabe-Kanal 15 mit einer Stimulationssignalausgangsschaltung 17 verbunden.

Die Schaltung zur bedarfsabhängigen Veränderung der Stimulationsrate 10 weist ferner eine mit der Steuer-und/oder Regelschaltung 3 gekoppelte Kommunikationseinheit 18 auf. Letztere dient neben der Programmierung des implantierten Herzschrittmachers der Übermittlung von Daten an externe Programmier- und Überwachungseinheiten.

Gesteuert vom Prozessor 11, wird über die am Speicher 6 ebenfalls angeschlossene Speicherbereichsauswahlschaltung 5 ein Speicherbereich angewählt. Nun wird dasjenige Zeitintervall (Zeitfenster) ausgegeben, bei dem der Unterschied für zwei verschiedene Belastungszustände des Patienten ein Maximum bildet. Gesteuert über den Prozessor 11. erfolgt dabei über die Auswahlschaltung 5 die Auswahl der Daten im Speicher 6.

Dazu ist es zuvor erforderlich, eine Meßreihe bei verschiedenen körperlichen Belastungen aufzunehmen, bei denen das jeweilige Zeitfenster variiert wird. Hierzu weist die Steuer- und/oder Regeleinrichtung 3 eine Schaltung 9 zur Auswahl des Zeitfensterabstandes Δtᵢ vom Bezugspunkt tₒ auf. Die Auswahlschaltungen 4. 5 und 9 enthalten entweder unabhängige, von der Steuer- und Regeleinrichtung 3 ansteuerbare Vor-/Rückwärtszähler oder sind in vorteilhafter Weise softwaremäßig in dem Prozessor 11 realisiert, der insbesondere die Meßwerte auswertet und die Bereiche mit maximaler Steigung Δκₘₐₓ automatisch für eine feste Einstellung (Speicherung im Speicher 6) findet.

Zunächst wird bei der niedrigsten Belastungsstufe Bₒ nacheinander in einzelnen Herzzyklen jeweils für unterschiedliche Zeitfenster der zugehörige erste Parameter Δκₒᵢⱼ ermittelt, wobei die Länge der Zeitintervalle Δtᵢ und Δtⱼ variiert wird. Bei der Belastungsstufe B₁ wird ein erstes Zeitfenster mit zugehörigem ersten Parameter Δκ₁ᵢⱼ im Speicher 6 abgespeichert. Tritt bei dieser Belastungsstufe ein Zeitfenster mit den Zeitintervallen Δtₘ und Δtₗ sowie ein zugehöriger erster Parameter auf, auf den die Ungleichung Δκ₁ₘₗ - Δκₒₘₗ > Δκ₁ᵢⱼ - Δκₒᵢⱼ zutrifft, wird dieses Zeitfenster Zᵢⱼ durch das neue Zeitfenster Zₘₗ mit dem neuen größeren Absolutwert der Differenz Δκ₁ₘₗ - Δκₒₘₗ der Werte des ersten Parameters überschrieben. Gleichzeitig wird der der Belastungsstufe B₁ zugehörige Wert Δκ₁ₘₗ im Speicher 6 gespeichert.

Einer Belastungsstufe Bₒ ist damit genau ein Zeitfenster Zᵢⱼ mit den Zeitintervallen Δtᵢ + Δtⱼ zugeordnet. So wird insbesondere unter Verwendung eines mit der Speicherbereichsauswahlschaltung 5 gekoppelten Prozessors 11 für mindestens eine weitere Belastungsstufe B₁ im Belastungsbereich dasjenige Zeitfenster in seiner relativen Lage zu einem festen Bezugspunkt im Herzyklus (Grenzen der Preejection-period) festgehalten, bei dem der Unterschied der Steigung des Integrals der Leitwerte ein Maximum bildet.

Dabei kommt es auf die absoluten Werte der Steigungen an, welche im durchlaufenen Bereich durchaus auch das Vorzeichen wechseln können. Auch ist vorteilhaft, daß nur Bereiche der in Figur 1 gezeigten Kurve mit monotoner Steigung berücksichtigt werden.

Auch kann der gesamte bei Belastungswechsel zu durchlaufende Bereich in eine Anzahl aufeinander folgender Belastungszonenbereiche unterteilt werden, denen Belastungsstufen zuordenbar sind. Zur n-ten Belastungsstute Bₙ wird dann genau ein Zeitfenster Zᵢⱼ mit den Zeitintervallen Δtᵢ und Δtⱼ ermittelt und dafür gesorgt, daß umschaltbare Zwischenbereiche für unterschiedliche Belastungszonenbereiche vorhanden sind.

Die Umschaltung der Beeinflussung des ersten durch den zweiten Parameter erfolgt hierbei beispielsweise mittels eines zusätzlich vorgesehenen Aktivitätssensors 12, der die Intensität der Bewegungen oder sonstigen körperlichen Tätigkeit erfaßt. Der Aktivitätssensor 12 ist mit dem Prozessor 11 über eine dritte Eingangsschaitung 13 der Steuer-und/oder Regeleinrichtung 3 verbunden. Der Prozessor 11 steuert über die Datenein-/ausgabe-Schaltung 15 für die jeweilige Belastungsstufe die Einspeicherung und den Abruf entweder eines Wertes Δκₘₐₓ bei fest vorgegebener unterer Leitwertsgrenze κᵢ oder der Grenzwerte κᵢ und κⱼ in den bzw. aus dem Speicher 6. Entsprechend den Grenzwerten gibt die Ratenauswahlschaltung 8 die Simulationsrate vor. Unter Verwendung des Prozessors 11 kann auch die Ratenauswahlschaltung 8 softwaremäßig realisiert werden.

Ein derartiger Aktivitätssensor 12 kann dabei auch zur Selbsteichung oder Kontrolle in der Weise dienen, daß bestimmt wird, welchen ermittelten Leitfähigkeitsänderungen bestimmte Stimulationsraten zuzuordnen sind.

Bei einer anderen bevorzugten Ausführungsform, ohne die dritte Eingangsschaltung, erfolgt die Umschaltung der Beeinflussung des ersten durch den zweiten Parameter durch das Ausgangssignal der Schaltung zur bedarfsabhängigen Veränderung der Stimulationsrate 10 selbst. Gegebenenfalls wird mit Hysterese dann umgeschaltet, wenn der Prozessor eine Steigung (Δκₘₐₓ im Zeitfenster) ermittelt, der bereits eine andere Betastungsstufe Bₙ₊₁ zuordenbar ist. Voraussetzung ist dafür, daß das System selbstadaptierend ist, d.h. während der Stimulation versucht wird, ob sich Bereiche größerer Steigungsänderung finden lassen.

Die Schaltungstechnik eines ratengesteuerten Schrittmachers kann als bekannt unterstellt werden, wobei es bei dieser Weiterbildung der vorliegenden Erfindung darauf ankommt, die Signalaufnahme des die Stimulationsrate bestimmenden Parameters zu verbessern. Dabei ist die Anwendung der Erfindung nicht davon abhängig, ob es sich dabei um eine open-loop oder closed-loop-Steuerung handelt.

## Patentansprüche

1. Herzschrittmacher mit einer Schaltung zur bedarfsabhängigen Veränderung der Stimulationsrate, wobei die zeitliche Änderung der elektrischen Leitfähigkeit im Herzen als erster Parameter ein Maß für die körperliche Belastung bzw. die erforderliche Pumpleistung des Herzens und damit eine Eingangsgröße für die Schaltung zur bedarfsabhängigen Veränderung der Stimulationsrate bildet,
**dadurch gekennzeichnet, dass**
die Differenz der elektrischen Leitfähigkeit zu Anfang und Ende eines einen zweiten Parameter darstellenden, im Rahmen einer Messreihe bei verschiedenen körperlichen Belastungszuständen jeweils variierten Zeitfensters mit einer relativen Lage zu einem festen Bezugszeitpunkt im Herzzyklus den ersten Parameter bildet,
dass dasjenige konkrete Zeitfenster innerhalb des Herzzyklus als Wert des zweiten Parameters in einem Speicher des Herzschrittmachers gespeichert wird,
bei dem die Differenz der in zwei unterschiedlichen Belastungszuständen des Patienten gemessenen Werte des ersten Parameters ein Maximum bildet, und dass die Schaltung Steuermittel umfasst, die zur Veränderung der Stimulationsrate in Abhängigkeit von dem ersten Parameter unter Beibehaltung des zuvor gespeicherten zweiten Parameters ausgebildet sind.

2. Herzschrittmacher nach Anspruch 1, **dadurch gekennzeichnet, daß** es sich bei den unterschiedlichen Belastungszuständen um einen Ruhezustand, d.h. Bereich kleiner körperlicher Belastung, und einen Arbeitszustand, d.h. Bereich relativ groBer körperlicher Belastung, handelt.

3. Herzschrittmacher nach Anspruch 2, **dadurch gekennzeichnet, daß** auch Steigungen unterschiedlichen Vorzeichens berücksichtigt werden.

4. Herzschrittmacher nach einem der vorangehenden Ansprüche, **dadurch gekennzeichnet, daß** nur Bereiche monotoner Steigung berücksichtigt werden.

5. Herzschrittmacher nach einem der Ansprüche 2 bis 4, **dadurch gekennzeichnet, daß** Umschaltmittel zwischen verschiedenen Belastungszuständen vorgesehen sind.

6. Herzschrittmacher nach einem der Ansprüche 2 bis 5, **dadurch gekennzeichnet, daß** die Umschaltung zwischen Bereichen unterschiedlicher Belastung gesteuert durch einen Aktivitätssensor erfolgt.

7. Herzschrittmacher nach einem der Ansprüche 2 bis 6, **dadurch gekennzeichnet daß** die Umschaltung zwischen Bereichen unterschiedlicher Belastung durch das Ausgangssignal der Schaltung zur belastungsabhängigen Veränderung der Stimulationsrate selbst, gegebenenfalls mit Hysterese, erfolgt.

## Claims

1. A cardiac pacemaker comprising a circuit for changing the stimulation rate as required, wherein the time change in electrical conductivity in the heart, as the first parameter, forms a measure of the physical strain and of the necessary pumping action of the heart and thus forms an input value for the circuit for changing the stimulation rate as required,
**characterised in that**
the difference in electrical conductivity at the beginning and end of a time window, respectively varied within a series of measurements with differing physical strain conditions and representing a second parameter, with a relative position to a fixed reference point in time in the cardiac cycle, forms the first parameter, and that
that concrete time window within the cardiac cycle is stored as the value of the second parameter in a memory of the cardiac pacemaker,
in which the difference of the measured values of the first parameter in two differing physical strain conditions of the patient forms a maximum, and **in that** the circuit comprises control means which are configured for changing the stimulation rate in dependence on the first parameter while maintaining the previously stored second parameter.

2. The cardiac pacemaker of claim 1, **characterised in that** the different states of strain are a resting state, i.e. a range of low physical strain, and a working state, i.e. a range of relatively high physical strain.

3. The cardiac pacemaker of claim 2, **characterised in that** gradients with a different preceding sign are also taken into account.

4. The cardiac pacemaker of any of the preceding claims, **characterised in that** only ranges with a monotonic gradient are taken into account.

5. The cardiac pacemaker of any of claims 2 to 4, **characterised in that** means are provided for switching over between different states of strain.

6. The cardiac pacemaker of any of claims 2 to 5, **characterised in that** the switchover between ranges of different strain takes place under the control of an activity sensor.

7. The cardiac pacemaker of any of claims 2 to 6, **characterised in that** the switchover between ranges of different strain takes place, possibly with hysteresis, by means of the output signal from the circuit for changing the actual stimulation rate dependent on the strain.

## Revendications

1. Stimulateur cardiaque avec un circuit pour la variation en fonction des besoins de la fréquence de stimulation, dans lequel la variation temporelle de la conductivité électrique dans le coeur en tant que premier paramètre constitue une mesure de la charge corporelle ou de la puissance de pompage du coeur nécessaire et donc une grandeur d'entrée pour le circuit pour la variation en fonction des besoins de la fréquence de stimulation, **caractérisé en ce que** la différence de conductivité électrique au début et à la fin d'une fenêtre temporelle représentant un second paramètre, qui est amené à varier dans le cadre d'une série de mesures dans différents états de charge corporels avec une position relative à un instant de référence fixe dans le cycle cardiaque forme le premier paramètre, **en ce que** la fenêtre temporelle concrète à l'intérieur du cycle cardiaque pour laquelle la différence des valeurs du premier paramètre mesurées dans deux états de charge différents du patient forme un maximum est mémorisée en tant que valeur du second paramètre dans une mémoire du stimulateur cardiaque pour laquelle la différence des valeurs du premier paramètre mesuré dans deux états de charge du patient différents forme un maximum et **en ce que** le circuit comprend des moyens de commande pour modifier la fréquence de stimulation en fonction du premier paramètre avec maintien du second paramètre mémorisé auparavant.

2. Stimulateur cardiaque selon la revendication 1, **caractérisé en ce qu'**il s'agit, concernant les différents états de charge, d'un état de repos, c'est-à-dire d'un domaine de faible charge coporelle, et d'un état de travail, c'est-à-dire d'un domaine de charge corporelle relativement importante.

3. Stimulateur cardiaque selon la revendication 2, **caractérisé en ce que** même des pentes de signe différent sont prises en compte.

4. Stimulateur cardiaque selon l'une des revendications précédentes, **caractérisé en ce que** seuls des domaines de pente monotone sont pris en compte.

5. Stimulateur cardiaque selon l'une des revendications 2 à 4, **caractérisé en ce qu'**il est prévu des moyens de commutation entre différents états de charge.

6. Stimulateur cardiaque selon l'une des revendications 2 à 5, **caractérisé en ce que** la commutation entre des domaines de charge différente est réalisée de manière commandée par un détecteur d'activité.

7. Stimulateur cardiaque selon l'une des revendications 2 à 6, **caractérisé en ce que** la commutation entre des domaines de charge différente est réalisée par le signal de sortie du circuit pour la variation en fonction de la charge de la fréquence de stimulation proprement dit, éventuellement avec hystérésis.
